# EUROPÄISCHE PATENTSCHRIFT

(11) **EP 0 914 625 B1**
(45) Veröffentlichungstag und Bekanntmachung des Hinweises auf die Patenterteilung: **17.10.2001**
(21) Anmeldenummer: 97936566.5
(22) Anmeldetag: 28.07.1997
(51) Int. Cl.: G02B 23/24

(54) **ENDOSKOP MIT WENIGSTENS EINEM GEKLEBTEN UND ZUSÄTZLICH GELÖTETEN ENDFENSTER**
ENDOSCOPE WITH AT LEAST ONE GLUED AND ADDITIONALLY WELDED END WINDOW
ENDOSCOPE AVEC AU MOINS UNE FENETRE TERMINALE COLLEE ET EN OUTRE SOUDEE

(30) Priorität: 26.07.1996 DE 19630090; 28.10.1996 DE 19644729
(43) Veröffentlichungstag der Anmeldung: 12.05.1999
(73) Patentinhaber: Karl Storz GmbH & Co. KG, 78532 Tuttlingen (DE)
(72) Erfinder: RUDISCHHAUSER, Jürgen, D-78532 Tuttlingen (DE)
(74) Vertreter: Münich, Wilhelm, Dr.
(86) Internationale Anmeldenummer: DE9701591
(87) Internationale Veröffentlichungsnummer: WO9804948

(56) Entgegenhaltungen:
- WO-A-95/24857
- DE-A- 3 740 417
- DE-U- 9 412 590
- US-A- 4 916 534
- US-A- 5 536 244

## Beschreibung

### Technisches Gebiet

Die Erfindung bezieht sich auf ein Endoskop gemäß dem Oberbegriff des Patentanspruchs 1.

### Stand der Technik

Derartige Endoskope sind sowohl in starrer als auch in flexibler Ausführung allgemein bekannt und werden sowohl zur Beobachtung von Hohlräumen beispielsweise in Motoren, Turbinen als auch in der Medizin zur Diagnose oder zur Durchführung von therapeutischen Eingriffen eingesetzt.

Da Endoskope im Einsatz häufig in mit einer - häufig aggressiven - Flüssigkeit gefüllte Hohlräume oder in Hohlräumen mit hoher Luftfeuchtigkeit eingesetzt werden, ist es erforderlich, daß das distale Endfenster das Außenrohr, in dem die Endoskopoptik angeordnet ist, fluid- und gasdicht abschließt.

An die fluid- und gasdichte Verbindung des Endfensters mit dem Außenrohr werden dabei hohe Anforderungen gestellt, da sich das Außenrohr mit dem Endfenster nicht nur in agressiven Medien befinden kann, sondern auch hohen Temperaturen ausgesetzt sein kann:

Insbesondere bei medizinischen Endoskopen wird die Verbindung des Endfensters mit dem Außenrohr durch das Einlegen in eine Sterilisierlösung und/oder durch eine Sterilisierung mit heißem Wasserdampf im Autoklaven bei ca. 140°C stark beansprucht. Beim Sterilisiervorgang wird in gleicher Weise wie das distale Endfenster auch das proximal angeordnete Okularfenster beansprucht.

Bei den meisten bekannten Endoskopen sind die Endfenster in das Außenrohr bzw. den Okulartrichter eingeklebt. Es hat sich jedoch gezeigt, daß Klebeverbindungen gerade dann, wenn sie einer Temperaturwechselbelastung und zusätzlich aggressiven Medien ausgesetzt werden, altern, so daß sie undicht werden, und nach einer gewissen Betriebszeit Feuchtigkeit und/oder Fluide in das Endoskop eindringen können.

Deshalb ist in der DE 37 40 417A1 vorgeschlagen worden, auf die Umfangsfläche des distalen Endfensters einen Metallfilm aufzubringen und diesen Metallfilm mit dem in der Regel aus einem nichtrostenden Metall bestehenden Außenrohr zu verlöten.

Hierzu ist eine sogenannte "Spaltlötung" erforderlich. Die sich bei einem Spalt-Lötvorgang ergebende Lötschicht kann jedoch nur sehr aufwendig auf Fehlstellen etc. kontrolliert werden, so daß es bei der Serienherstellung möglich ist, daß Fehler in der Lötverbindung übersehen werden. Dies hat zur Folge, daß nach einer bestimmten Zeit das Endfenster das Endoskop nicht mehr fluiddicht verschließt.

### Darstellung der Erfindung

Der Erfindung liegt die Aufgabe zugrunde, ein Endoskop gemäß dem Oberbegriff des Patentanspruchs 1 derart weiterzubilden, daß das bzw. die Endfenster sicher und in leicht kontrollierbarer Weise mit dem Außenrohr bzw. dem Okulartrichter fluiddicht verbunden sind.

Eine erfindungsgemäße Lösung dieser Aufgabe ist im Patentanspruch 1 angegeben. Weiterbildungen der Erfindung sind Gegenstand der Ansprüche 2ff.

Erfindungsgemäß sind das distale Endfenster und/oder das Okular mit ihrem Umfangsrand in an sich bekannter Weise in das Außenrohr bzw. den Okulartrichter oder ein in das Außenrohr bzw. den Okulartrichter eingesetztes Zwischenstück eingeklebt. Zusätzlich ist auf wenigstens eine Endfläche des oder der Fenster, bevorzugt die dem Inneren zugekehrte Endfläche eine lötbare ringförmige Schicht aufgebracht, über die das Fenster zusätzlich durch eine Lotschicht mit dem Außenrohr, dem Okulartrichter oder dem Zwischenstück verbunden ist.

Erfindungsgemäß sind damit zwei voneinander unabhängige Verbindungen vorgesehen, so daß selbst bei einer Beschädigung oder einer nichtordnungsgemäßen Ausführung der einen Verbindung das Endoskop auch bei starker Beanspruchung fluid- und gasdicht bleibt.

Darüberhinaus wird auch die chemische Resistenz im Vergleich zu bekannten Endoskopen erhöht.

Dabei ist die in an sich bekannter Weise vorgesehene Spaltklebung im Vergleich zu einem Spalt-Lötvorgang vergleichsweise einfach durchzuführung und zu kontrollieren. Die zusätzlich vorgesehene Lötverbindung, die im Gegensatz zu der aus der DE 37 40 417 A1 vorgeschlagenen Spalt-Lötverbindung auf eine Außenfläche aufgebracht wird, ist nicht nur leicht auszuführen, sondern das Lötergebnis ist auch visuell einfach und sicher zu kontrollieren.

Zur Herstellung der erfindungsgemäßen Verbindung wird bevorzugt erst die Lötverbindung ausgeführt. Anschließend erfolgt die Spaltklebung. Im Falle von Lötverbindungen auf beiden Endflächen wird bevorzugt erst die Lötverbindung auf der proximalen, d.h. der dem Innenraum zugekehrten Endfläche, anschließend die Klebung und dann die Lötverbindung auf der distalen Endfläche ausgeführt. Durch diese Vorgehensweise lassen sich die einzelnen Verbindungen einfach visuell kontrollieren.

In jedem Falle hat die erfindungsgemäße Verbindung den Vorteil, daß das Endfenster im Vergleich zum Stand der Technik dünner ausgeführt werden kann, ohne daß die Verbindung zwischen Endfenster und Außenrohr schlechter wird.

Durch den Einsatz zweier unterschiedlicher Verbindungstechniken erhält man gerade bei thermisch induzierten Spannungen eine günstigere Kraftweiterleitung als beim Einsatz lediglich einer Verbindungstechnik.

Im Falle der Verwendung eines Zwischenstückes kann dieses mit dem Außenrohr bzw. dem Okulartrichter auf die verschiedensten Arten verbunden bzw. befestigt werden. Insbesondere ist es dabei möglich, außer mit bekannten Verbindungstechniken das Zwischenstück im Außenrohr bzw. Okulartrichter in gleicher Weise wie das Fenster im Zwischenstück erfindungsgemäß zu befestigen.

Die erfindungsgemäße Verbindung eignet sich für Endoskope für beliebige Anwendungsfälle und beliebige Blickwinkel.

Weiterbildungen der Erfindung sind Gegenstand der Ansprüche 2ff.:
Die lötbare Schicht, die auf eine Endfläche des Endfensters aufgebracht wird, ist bevorzugt eine Metallschicht, die aufgedampft oder beispielsweise durch Tauchen oder elektrolytisch abgeschieden wird.
Die Schmelztemperatur des eingesetzten Lotes und die Erweichungstemperatur des Klebstoffes können dabei je nach Einsatzgebiet des Endoskops gewählt werden. Im Falle medizinischer Endoskope, die durch Autoklavieren sterilisiert werden sollen, müssen die genannten Temperaturen natürlich so hoch sein, daß die Verbindungsschichten durch die beim Autoklaviervorgang verwendeten Temperaturen nicht geschädigt werden.
Dies ist beispielsweise dann der Fall, wenn als Lot ein "deutlich über der Autoklaviertemperatur (ca. 140°C) schmelzendes Lot und als Klebstoff ein UV-härtbarer Klebstoff verwendet wird.
Das distale Endfenster kann in bekannter Weise bei spielsweise aus Quarzglas, BK 7 oder einem Saphirmaterial bestehen.
Die erfindungsgemäße Vorgehensweise, die Lötschicht nicht auf die Umfangsfläche wie beim Stand der Technik, sondern auf eine oder beide Endflächen aufzubringen, hat den weiteren Vorteil, daß die hergestellte ringförmige Schicht gleichzeitig als Aperturblende oder als Streulichtblende des Endoskopobjektivs dienen kann.

### Kurze Beschreibung der Zeichnung

Die Erfindung wird nachstehend anhand eines Ausführungsbeispiels unter Bezugnahme auf die Zeichnung näher beschrieben, in der zeigen:
- Fig. 1: einen Längsschnitt durch den distalen Endbereich, und
- Fig. 2: eine Aufsicht auf den distalen Endbereich eines erfindungsgemäßen Endoskops.

### Darstellung eines Ausführungsbeispiels

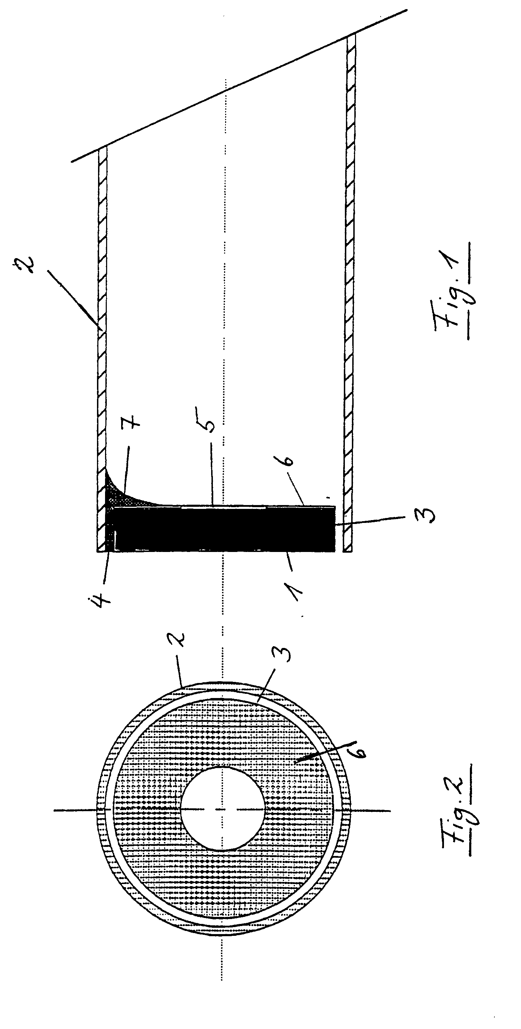

Fig. 1 zeigt einen Längsschnitt durch den distalen Endbereich eines Endoskops. Mit dem Bezugszeichen 1 ist ein distales Endfenster bezeichnet, das beispielsweise aus Quarzglas, BK 7 oder einem Saphirmaterial bestehen kann. Das Endfenster ist in ein Außenrohr 2 eingesetzt, das die nicht dargestellte Endoskopoptik aufnimmt, also ein Objektiv und einen Bildweiterleiter, der eine Reihe von Relaylinsensystemen oder ein optisches Faserbündel aufweisen kann. Das Außenrohr besteht beispielsweise aus Monel oder einem VA-Stahl geeigneter Korrosionsbeständigkeit.

Zwischen der Umfangsfläche 3 des Endfensters 1 und dem Außenrohr 2 befindet sich eine Klebstoffschicht 4, die insbesondere aus einem UV-härtbaren Klebstoff bestehen kann. Zusätzlich ist auf die proximale Endfläche 5 des Endfensters 1 eine ringförmige Schicht 6 aufgebracht, die lötbar ist und insbesondere eine Metallschicht sein kann. Das Endfenster 1 ist über die lötbare ringförmige Schicht 6 durch eine Lötverbindung 7 mit dem Außenrohr 2 verbunden.

Fig. 2 zeigt eine Aufsicht auf Endfenster, in der deutlich der Spalt zwischen dem Endfenster 1 und dem Außenrohr 2 zu sehen ist, der mit Klebstoff ausgefüllt wird.

Im Falle des Okularfensters wird entsprechend vorgegangen.

Der erfindungsgemäße Grundgedanke, zusätzlich zu einer Umfangs-Klebung eine stirnseitige Lotverbindung vorzusehen, kann bei flexiblen oder starren Endoskopen eingesetzt werden. Besonders vorteilhaft ist er jedoch bei starren Endoskopen, da diese über ein lötbares Außenrohr verfügen, das zumeist aus Monell besteht. Bei flexiblen Endoskopen kann gegebenenfalls ein Zwischenstück erforderlich sein.

## Patentansprüche

1. Endoskop mit einem Außenrohr, in dessen distalem Endbereich ein Endoskopobjektiv, dessen Bild ein Bildweiterleiter zu dem proximalen Ende des Endoskops leitet, und ein distales Endfenster vorgesehen sind, das das Außenrohr fluid- und gasdicht abschließt, und durch das der Strahlengang des Endoskopobjektivs verläuft, wobei das distale Endfenster und/oder das Okularfenster mit ihrem Umfangsrand in an sich bekannter Weise in das Außenrohr bzw. den Okulartrichter oder ein in das Außenrohr bzw. den Okulartrichter eingesetztes Zwischenstück eingeklebt ist,
**dadurch gekennzeichnet, daß** auf wenigstens einer Endfläche des Endfensters eine lötbare ringförmige Schicht aufgebracht ist, über die das Endfenster zusätzlich durch eine Lotschicht mit dem Außenrohr oder dem Zwischenstück verbunden ist.

2. Endoskop nach Anspruch 1,
**dadurch gekennzeichnet, daß** die lötbare ringförmige Schicht auf die Innenfläche aufgebracht ist.

3. Endoskop nach Anspruch 1,
**dadurch gekennzeichnet, daß** auf beide Endflächen lötbare ringfömige Schichten aufgebracht sind.

4. Endoskop nach einem der Ansprüche 1 bis 3,
**dadurch gekennzeichnet, daß** die lötbare Schicht eine Metallschicht ist, die aufgedampft oder beispielsweise durch Tauchen oder elektrolytisch abgeschieden ist.

5. Endoskop nach einem der Ansprüche 1 bis 4,
**dadurch gekennzeichnet, daß** die Schmelztemperatur des Lotes und die Erweichungstemperatur des Klebstoffes so hoch sind, daß das Endoskop durch Autoklavieren sterilisierbar ist.

6. Endoskop nach einem der Ansprüche 1 bis 5,
**dadurch gekennzeichnet, daß** das Lot ein Hartlot ist.

7. Endoskop nach Anspruch 5 oder 6,
**dadurch gekennzeichnet, daß** der Klebstoff ein UV-härtbarer Klebstoff ist.

8. Endoskop nach einem der Ansprüche 1 bis 7,
**dadurch gekennzeichnet, daß** das Endfenster aus Quarzglas oder BK7 besteht.

9. Endoskop nach einem der Ansprüche 1 bis 8,
**dadurch gekennzeichnet, daß** das Endfenster aus einem Saphir besteht.

10. Endoskop nach einem der Ansprüche 1 bis 9,
**dadurch gekennzeichnet, daß** die auf eine Endfläche des Endfensters aufgebrachte ringförmige Schicht die Aperturblende oder eine Streulichtblende des Endoskopobjektivs bildet.

11. Endoskop nach einem der Ansprüche 1 bis 10,
**dadurch gekennzeichnet, daß** das Endfenster in an sich bekannter Weise eine optische Wirkung hat.

12. Endoskop nach einem der Ansprüche 1 bis 11,
**dadurch gekennzeichnet, daß** das Außenrohr aus einem nichtrostendem Metall, wie Monel besteht.

## Claims

1. Endoscope comprising an external tube having a distal end section where an endoscope lens producing an image that is transferred by a video conductor to the proximal end of the endoscope, and a distal end window are provided that closes the external tube in a fluid-tight and gas-tight manner and through which the beam path of the endoscope lens is passing, wherein, in a manner known per se, the distal end window and/or the eyepiece window is inserted into and adhesively connected by its peripheral edge to said external tube or the eyepiece funnel, respectively, or an adapter inserted into said external tube or said eyepiece funnel, respectively,
**characterised in that** a brazable annular layer is applied on at least one end surface of said end window, via which said end window is additionally connected by a brazing filler metal layer to said external tube or said adapter.

2. Endoscope according to Claim 1,
**characterised in that** said brazable annular layer is applied onto the internal surface.

3. Endoscope according to Claim 1,
**characterised in that** brazable annular layers are applied on both end surfaces.

4. Endoscope according to any of the Claims 1 to 3,
**characterised in that** said brazable layer is a metal layer which is evaporated or deposited, for instance by immersion or electrolysis.

5. Endoscope according to any of the Claims 1 to 4,
**characterised in that** the fusing temperature of said brazing filler metal and the softening temperature of said adhesive are so high that the endoscope may be sterilised by autoclaving.

6. Endoscope according to any of the Claims 1 to 5,
**characterised in that** said brazing filler metal is a brazing solder.

7. Endoscope according to Claim 5 or 6,
**characterised in that** said adhesive is an adhesive that can be set under UV light.

8. Endoscope according to any of the Claims 1 to 7,
**characterised in that** said end window consists of quartz glass or BK 7.

9. Endoscope according to any of the Claims 1 to 8,
**characterised in that** said end window consists of a sapphire.

10. Endoscope according to any of the Claims 1 to 9,
**characterised in that** said annular layer applied on one end surface of said end window constitutes the aperture plate or a scattered-light diaphragm of the endoscope lens.

11. Endoscope according to any of the Claims 1 to 10,
**characterised in that** said end window produces an optical effect in a manner known per se.

12. Endoscope according to any of the Claims 1 to 11,
**characterised in that** said external tube consists of a stainless metal such as monel metal.

## Revendications

1. Endoscope comprenant un tube extérieur à une section terminale distale, à laquelle sont disposés un objectif de l'endoscope, qui produit une image transférée par un conducteur vidéo à la section terminale proximale de l'endoscope, et une fenêtre terminale à l'extrémité distale, qui ferme le tube extérieur de façon étanche aux liquides et aux gaz et par lequel passé la marche des rayons, dans lequel, d'une façon connue en soi, la fenêtre terminale à l'extrémité distale et/ou la fenêtre de l'oculaire est introduite dans et collée, par son bord périphérique, audit tube extérieur ou respectivement à la tulipe de l'oculaire ou à un adaptateur inséré dans ledit tube extérieur ou respectivement dans ladite tulipe de l'oculaire,
**caractérisé en ce qu'**une couche annulaire brasable est appliquée sur au moins une surface terminale de ladite fenêtre terminale, via laquelle ladite fenêtre terminale est, au surplus, reliée, moyennant une couche en un métal d'apport de brasage, audit tube extérieur ou audit adaptateur.

2. Endoscope selon la revendication 1,
**caractérisé en ce que** ladite couche annulaire brasable est appliquée sur la surface intérieure.

3. Endoscope selon la revendication 1,
**caractérisé en ce que** des couches annulaires brasables sont appliquées sur les deux faces terminales.

4. Endoscope selon une quelconque des revendications 1 à 3,
**caractérisé en ce que** ladite couche brasable est une couche en métal, qui est métallisée sous vide ou déposée, par exemple, par immersion ou par électrolyse.

5. Endoscope selon une quelconque des revendications 1 à 4,
**caractérisé en ce que** la température de fusion dudit métal d'apport de brasage et la température d'amollissement dudit agent adhésif sont hautes afin de permettre la stérilisation de l'endoscope par autoclavage.

6. Endoscope selon une quelconque des revendications 1 à 5,
**caractérisé en ce que** ledit métal d'apport de brasage est un métal d'apport de brasage fort.

7. Endoscope selon la revendication 5 or 6,
**caractérisé en ce que** ledit agent adhésif est une substance durcissable moyennant des rayons UV.

8. Endoscope selon une quelconque des revendications 1 à 7,
**caractérisé en ce que** ladite fenêtre terminale consiste en verre quartzeux ou BK 7.

9. Endoscope selon une quelconque des revendications 1 à 8,
**caractérisé en ce que** ladite fenêtre terminale consiste en un saphir.

10. Endoscope selon une quelconque des revendications 1 à 9,
**caractérisé en ce que** ladite couche annulaire, qui est appliquée sur une face terminale de ladite fenêtre terminale, constitue le diaphragme d'ouverture ou un diaphragme de lumière diffusée de l'objectif de l'endoscope.

11. Endoscope selon une quelconque des revendications 1 à 10,
**caractérisé en ce que** ladite fenêtre terminale crée un effet optique d'une façon connue en soi.

12. Endoscope selon une quelconque des revendications 1 à 11,
**caractérisé en ce que** ledit tube extérieur consiste en un métal antirouille, par exemple en monel.
